# EUROPEAN PATENT SPECIFICATION

(11) **EP 3 446 677 B1**
(45) Date of publication and mention of the grant of the patent: **07.08.2024**
(21) Application number: 17819984.0
(22) Date of filing: 21.06.2017
(51) Int. Cl.: A61K 8/67, A61Q 5/02, A61Q 5/12, A61Q 5/00

(54) **DAMAGED HAIR-IMPROVING AGENT AND DAMAGED HAIR-IMPROVING COSMETIC CONTAINING SAME**
MITTEL ZUR VERBESSERUNG VON GESCHÄDIGTEM HAAR UND KOSMETIKUM ZUR VERBESSERUNG VON GESCHÄDIGTEM HAAR DAMIT
AGENT D'AMÉLIORATION DES CHEVEUX ABÎMÉS ET PRODUIT COSMÉTIQUE D'AMÉLIORATION DES CHEVEUX ABÎMÉS CONTENANT CELUI-CI

(30) Priority: 29.06.2016 JP 2016128304
(43) Date of publication of application: 27.02.2019
(73) Proprietor: Nikko Chemicals Co., Ltd., Tokyo 103-0002 (JP); Nippon Surfactant Industries Co., Ltd., Tokyo 103-0002 (JP)
(72) Inventor: MISONO Takeshi, Tokyo 174-0046 (JP); USHIJIMA Shodai, Tokyo 174-0046 (JP); YAMAGUCHI Shunsuke, Tokyo 174-0046 (JP)
(74) Representative: Gille Hrabal Partnerschaftsgesellschaft mbB Patentanwälte
(86) International application number: PCT/JP2017/022838
(87) International publication number: WO 2018/003625

(56) References cited:
- FR-A1- 3 015 244
- JP-A- 2001 354 522
- JP-A- 2003 040 747
- JP-A- 2010 235 570
- JP-A- 2013 018 720
- JP-A- 2014 114 291
- JP-A- 2014 152 168
- JP-A- H0 578 229
- JP-A- H10 510 523
- JP-A- S61 183 206
- JP-A- S62 142 108
- DATABASE GNPD [online] MINTEL; 12 October 2011 (2011-10-12), ANONYMOUS: "Instant Restorative Hair Serum", XP055641400, retrieved from www.gnpd.com Database accession no. 1651322
- DATABASE GNPD [online] MINTEL; 24 April 2015 (2015-04-24), ANONYMOUS: "Hair Treatment II", XP055641409, retrieved from www.gnpd.com Database accession no. 3154071
- DATABASE GNPD [online] MINTEL; 10 December 2015 (2015-12-10), ANONYMOUS: "Mask", XP055641420, retrieved from www.gnpd.com Database accession no. 3614857

## Description

### Field of the Invention

The present invention relates to damaged hair improving agents having excellent improving effects on damaged hair and damaged hair improving cosmetics containing the same.

### Background of the Invention

Keeping hair well-groomed is of great interest regardless of sex, and in recent years hair arrangements to make hair look attractive have been practiced such as coloring to change the color of hair by chemical treatment, perms, straight perms and curly hair correction to change the shape of hair, brushing using a comb in the daily life, and styling with a hair arrangement device used by generating heat (blow dryer, hair iron, etc.). However, such practices are a factor to induce hair damages, reduce firmness, body and glossiness of hair, thereby causing split ends, broken hair, dry hair, or unmanageable hair. Additional examples of the factor for damaging hair include ultraviolet rays from the sunlight.

For the purpose of improving damaged hair, reports have been made on hair cosmetics for repairing or preventing damaged hair by using 18-methyleicosanoic acid and a tertiary amine compound in combination (JP-B 5094216, JP-B 4469874, JP-B 4469875, and JP-B 4559392) and hair cosmetics for improving hair waves and tangles by containing lactone derivatives (JP-B 5726469, JP-A 2014-65688, and JP-A 2013-53113). However, for 18-methyleicosanoic acid used in these hair cosmetics, for example, the synthesis method described in JP-B 5252905 is cumbersome, making it difficult for mass production, thus hardly available and extremely expensive, hence problematic. The lactone derivative does not demonstrate the improving effect unless heat treatment is carried out after applied to hair, hence problematic.

Patent application FR-A3015244 discloses hair care compositions containing ascorbic acid derivatives, wherein R is a saturated or unsaturated linear or branched hydrocarbon group having 11 to 21 carbon atoms, and X an O atom or a group -NH.

### Summary of the Invention

The present invention has an object to provide damaged hair improving agents and damaged hair improving cosmetics, capable of providing excellent improving effects on damaged hair without using cumbersome work, special components, and expensive components and retaining the effects for an extended period of time.

The present inventors conducted extensive studies on damaged hair improving agents and damaged hair improving cosmetics containing the same and found that one or more selected from L-ascorbic acid derivatives represented by general formula (1) and salts thereof have excellent improving effects on damaged hair, whereby the present invention was accomplished.

The present invention relates to the use of a damaged hair improving agents containing, as a main component, one or more selected from L-ascorbic acid derivatives represented by general formula (1) and salts thereof: 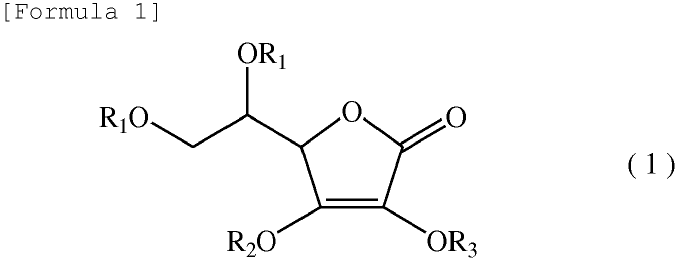 wherein R₁ is each a linear or branched saturated or unsaturated fatty acid residue having 8 to 36 carbon atoms, R₂ is a linear or branched saturated or unsaturated fatty acid residue having 8 to 36 carbon atoms, R₃ is a linear or branched saturated or unsaturated fatty acid residue having 8 to 36 carbon, and wherein this derivative is present in an amount of from 0.5 to 20% by mass; for increasing the hydrophobicity of the hair surface and/or making hair smooth and/or further enhancing glossiness.

The damaged hair improving agents containing, as a main component, one or more selected from L-ascorbic acid derivatives represented by general formula (1) and salts thereof and the damaged hair improving cosmetics containing the same can achieve improving effects on damaged hair when applied to damaged hair and further retain the effects for an extended period of time when heat-treated by a method using a blow dryer after application.

The one or more selected from the L-ascorbic acid derivatives represented by general formula (1) and salts thereof for use in the present invention have been conventionally used in cosmetics other than hair cosmetics as useful components due to physiological activities such as a skin-whitening effect and an anti-aging effect by delayed melanin production, lipid peroxide prevention, and collagen production promotion. The present inventors found that one or more selected from the L-ascorbic acid derivatives represented by general formula (1) and salts thereof have effects of improving the surface of damaged hair in addition to the skin-whitening effect and the anti-aging effect, based on which the present invention provides damaged hair improving agents having excellent effects of improving damaged hair and damaged hair improving cosmetics containing the same.

When the damaged hair improving agents containing one or more selected from the L-ascorbic acid derivatives represented by general formula (1) and salts thereof and the damaged hair improving cosmetics containing the same according to the present invention are applied to hair, improving effects are achieved such as increasing the hydrophobicity of the hair surface, making hair smooth, and further enhancing glossiness, and these effects are fully demonstrated without heat treatment. When heat treatment is further added, these improving effects are retained for an extended period of time.

### Embodiments of the Invention

The damaged hair improving agents for use in of the present invention contains, as a main component, one or more selected from L-ascorbic acid derivatives represented by general formula (1) and salts thereof: 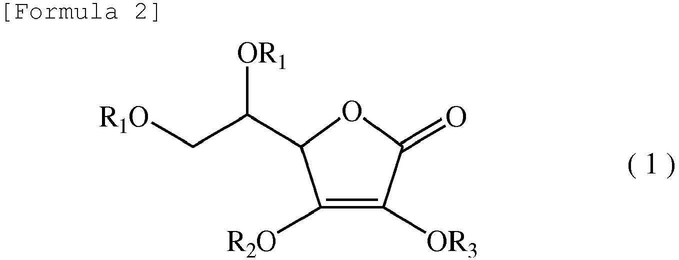 wherein R₁ is each independently a linear or branched saturated or unsaturated fatty acid residue having 8 to 36 carbon atoms, R₂ is a linear or branched saturated or unsaturated fatty acid residue having 8 to 36 carbon atoms, R₃ is a linear or branched saturated or unsaturated fatty acid residue having 8 to 36 carbon atoms.

For R₁ to R₃ in the L-ascorbic acid derivatives represented by general formula (1) and salts thereof, the linear or branched saturated or unsaturated fatty acid residue has 8 to 36 carbon atoms, and preferably the linear or branched saturated or unsaturated fatty acid residue has 12 to 22 carbon atoms. The fatty acid residue refers to a residue obtained by removing a hydroxyl group from a carboxyl group of a linear or branched saturated or unsaturated fatty acid, and the fatty acid is a saturated fatty acid or unsaturated fatty acid and examples include caprylic acid, capric acid, lauric acid, myristic acid, palmitic acid, hexyldecanoic acid, oleic acid, linoleic acid, arachidic acid, and behenic acid.

Examples of the salt of the L-ascorbic acid derivatives include sodium, potassium and magnesium salts.

The L-ascorbic acid derivatives represented by general formula (1) and salts thereof for use in the present invention are easily synthesized as described in JP-B 3253735, further widely blended in cosmetics other than hair cosmetics, and readily available at a low cost.

The L-ascorbic acid derivatives and salts thereof are not particularly limited, and examples include ascorbyl tetrapalmitate and ascorbyl tetra-2-hexyldecanoate and one or two of these can be used. More preferable examples include ascorbyl tetra-2-hexyldecanoate (product of Nikko Chemicals, Co., Ltd., NIKKOL VC-IP).

Further, the one or more selected from the L-ascorbic acid derivatives represented by general formula (1) and salts thereof for use in the present invention have a feature of being soluble in oil components and water.

The one or more selected from the L-ascorbic acid derivatives represented by general formula (1) and salts thereof for use in the present invention can be directly used as the damaged hair improving agents, or blended in oil components or water, and preferably in oil components. In the damaged hair improving agents for use in the present invention, the amount of the one or more selected from the L-ascorbic acid derivatives represented by general formula (1) and salts thereof blended in oil components or water is 0.5 to 20% by mass.

The oil components used in the damaged hair improving agents for use in the present invention are not particularly limited, and examples include squalane, liquid paraffin, tri(caprylic acid/capric acid)glyceryl, triethylhexanoin, cetyl ethylhexanoate, diethyl sebacate, ethylhexyl palmitate, isopropyl myristate, olive oil, apricot kernel oil, sunflower oil, argan oil, jojoba oil, silicone oil, and dimethyl silicone oil, and one or two or more of these can be used. Examples of the oil component preferably include squalane, tri(caprylic acid/capric acid)glyceryl, and cetyl ethylhexanoate.

In the damaged hair improving cosmetics containing the damaged hair improving agents for use in the present invention, various components used in typical hair cosmetics such as oil components, higher alcohols, fatty acids, polar lipids, moisturizing components, antibacterial components, viscosity adjusting agents, UV absorbers, pigments, or perfumes can be blended within a range where the effects of the present invention are not affected.

Examples of the oil components include hydrocarbons such as squalane, liquid paraffin, vegetable oils such as olive oil, macadamia nut oil, and jojoba oil, animal oils such as beef tallow, esters such as triethylhexanoin, tri(caprylic acid/capric acid)glyceryl, cetyl ethylhexanoate, diethyl sebacate, and ethylhexyl palmitate, silicones such as dimethyl silicone, phenylmethyl silicone, and cyclomethicone.

Examples of the higher alcohols include lauryl alcohol, cetyl alcohol, stearyl alcohol, isostearyl alcohol, behenyl alcohol, myristyl alcohol, oleyl alcohol, and cetostearyl alcohol. Examples of the fatty acid include linear fatty acids, branched fatty acids, unsaturated fatty acids, and hydroxy fatty acids having 12 or more carbon atoms. Examples of the polar lipids include ceramides, phospholipids, cholesterols and derivatives thereof, and glycolipids. Examples of the moisturizing components include polyethylene glycol, glycerin, sorbitol, xylitol, maltitol, chondroitin sulfate, and hyaluronic acid.

Further, the purpose of use of the damaged hair improving cosmetics according to the present invention is not particularly limited, and examples include hair cosmetics such as hair oil, hair cream, hair milk, hair liquid, hair lotion, hair tonic, hair conditioner, hair rinse, hair treatment, hair shampoo, hair mousse, set gel, pomade, stick pomade, set lotion, regardless of whether they are oil-based, emulsion-based, or waterbased.

The damaged hair improving agents of the present invention can be readily blended in any damaged hair improving cosmetics, and the amount blended thereof is 0.01 to 100% by mass, preferably 0.05 to 50.0% by mass, and more preferably 0.1 to 30.0% by mass.

### Examples

Hereinbelow, the present invention is specifically described in reference to Examples but the technical range of the present invention is not limited thereto. The amounts blended in the following products of the present invention and comparative products are expressed in % by mass.

### Example 1

### Water-repellency evaluation of hair

### 1. Experiment overview

Water-repellency evaluation of the hair surface was carried out using the damaged hair improving agents containing L-ascorbic acid derivatives represented by general formula (1) or salts thereof.

### 2. Experiment method

Each of the hair agents A to F shown in Table 1, containing L-ascorbic acid derivatives or salts thereof and an oil component or water, was prepared. The damaged hair improving agent A comprises a L-ascorbic acid derivative of formula (I).

Hair bundles made of healthy persons' hair (product of Beaulax Co., Ltd.) were bleached using hydrogen peroxide water and an ammonia aqueous solution to produce hair bundles of damaged hair.

After each of the damaged hair improving agents was applied to the damaged hair bundle produced and was settled throughout the entire damaged hair bundle, no heat treatment was carried out to some of the hair bundles whereas heat treatment was carried out to other hair bundles by blowing hot air for 5 minutes using a blow dryer. Regardless of carrying out heat treatment, the hair bundles were rinsed alternately with a 3% by mass sodium lauryl sulfate aqueous solution and water. This procedure was repeated three times and subsequently the moisture on the hair bundles was wiped off using a towel. The hair bundles obtained by this treatment were measured for the contact angle of water against the hair surface using an automatic contact angle meter (product of Kyowa Interface Science Co., Ltd., DropMaster, DM-501) .

### 3. Results

The experiment results are shown in Table 1. When the damaged hair improving agents, which is for use in the present invention product 1-1 containing the L-ascorbic acid derivatives represented by general formula (1) or salts thereof, were applied to the damaged hair, the contact angles of water against the hair surface became high, confirming the water-repellency of the damaged hair bundles having been enhanced. It means that the damaged hair bundles to which the damaged hair improving agents for use in the present invention products were applied were improved to healthy hair as the contact angle of water against healthy hair is supposed to be 90° or more. The damaged hair improving agents for use in the present invention products demonstrated the water-repellency effect without carrying out heat treatment, and the water-repellency was even more demonstrated when heat treatment was further carried out. Further, the water-repellency effect was demonstrated depending on the presence or absence of a fatty acid residue of the L-ascorbic acid derivatives and salts thereof, regardless of the damaged hair improving agents being water-soluble or oil-soluble.

**[Table 1]**

| | Present invention products | | | | Comparative products | | | |
|---|---|---|---|---|---|---|---|---|
| | 1-1 | 1-2 | 1-3 | 1-4 | 1-1 | 1-2 | 1-3 | 1-4 |
| Squalane | 99.0 | 99.0 | 99.0 | | | | 100.0 | |
| Purified water | | | | 990 | 99.0 | 99.0 | | 100.0 |
| A | 1.0 | | | | | | | |
| B | | 1.0 | | | | | | |
| C | | | 1.0 | | | | | |
| D | | | | 1.0 | | | | |
| E | | | | | 1.0 | | | |
| F | | | | | | 1.0 | | |
| Total | 100.0 | 100.0 | 100.0 | 100.0 | 100.0 | 100.0 | 100.0 | 100.0 |
| Contact angle (°) Not heat treated | 110 | 101 | 98 | 98 | 70 | 70 | 81 | 71 |
| Contact angle (°) Heat treated | 115 | 108 | 101 | 105 | 73 | 72 | 80 | 70 |

| | | | | | | | | |
|---|---|---|---|---|---|---|---|---|
| A: Ascorbyl tetra-2-hexyldecanoate B: Ascorbyl dipalmitate C: Ascorbyl palmitate D: Trisodium ascorbyl palmitate phosphate E: Magnesium ascorbyl phosphate F: Disodium ascorbyl sulfate | | | | | | | | |

### Example 2

### Sensory evaluation on hair

### 1. Experiment overview

The sensory evaluation was conducted on the damaged hair bundles treated with the damaged hair improving agents containing the L-ascorbic acid derivatives represented by general formula (1) or salts thereof.

### 2. Experiment method

Each of the damaged hair improving agents (the present invention product 2-1), containing 3% by mass of the L-ascorbic acid derivatives represented by general formula (1) shown in Table 2 or salts thereof and squalane as an oil component for the balance, was prepared. Additionally, squalane (100% by mass) was used as Comparative Product 2-1. Hair bundles made of healthy persons' hair (product of Beaulax Co., Ltd.) were bleached using hydrogen peroxide water and an ammonia aqueous solution to produce hair bundles of damaged hair. After each of the damaged hair improving agents was applied to the damaged hair bundle produced, and was settled throughout the entire damaged hair bundle, no heat treatment was carried out to some of the hair bundles whereas heat treatment was carried out to other hair bundles by blowing hot air for 5 minutes using a blow dryer. Regardless of carrying out heat treatment, the hair bundles were rinsed alternately with a 3% by mass sodium lauryl sulfate aqueous solution and water. This procedure was repeated three times and subsequently the moisture on the hair bundles was wiped off using a towel. Sensory evaluation on the obtained hair bundles was carried out by 5 subjects using the following evaluation criteria.

### 3. Results

The experiment results are shown in Table 2. When the damaged hair improving agents, which is the product 2-1 for use in the present invention containing one or more selected from the L-ascorbic acid derivatives represented by general formula (1) and salts thereof, were applied to the damaged hair, improvements in the sensory evaluations on finger runningness, glossy appearance, firmness and body were confirmed, and the effects were demonstrated without reduction even when heat treatment was carried out.

**[Table 2]**

| | Present invention products | | | | | | | | Comparative product | |
|---|---|---|---|---|---|---|---|---|---|---|
| | 2-1 | | 2-2 | | 2-3 | | 2-4 | | 2-1 | |
| Main component used | A | | B | | C | | D | | G | |
| Heat treatment | Not treated | Treated | Not treated | Treated | Not treated | Treated | Not treated | Treated | Not treated | Treated |
| Finger runningness | very good | very good | very good | very good | good | very good | good | good | poor | poor |
| Glossy appearance | very good | very good | very good | very good | good | good | good | good | poor | poor |
| Manageability | very good | very good | good | good | good | good | good | good | poor | poor |
| Dry ness | good | good | good | good | good | good | good | good | poor | poor |
| Firmness and Body | very good | very good | good | very good | good | good | good | good | poor | poor |

| | | | | | | | | | | |
|---|---|---|---|---|---|---|---|---|---|---|
| A: Ascorbyl tetra-2-hexyldecanoate B: Ascorbyl dipalmitate C: Ascorbyl palmitate D: Trisodium ascorbyl palmitate phosphate G: Squalane | | | | | | | | | | |

### Evaluation criteria

Notably improved compared to damaged hair before the damaged hair improving agent was applied: Very good
Improved compared to damaged hair before the damaged hair improving agent was applied: Good
Slightly improved compared to damaged hair before the damaged hair improving agent was applied: Fair
No change compared to damaged hair before the damaged hair improving agent was applied: Poor

### Example 3

### Confirmation on retainability of damaged hair improving effects

### 1. Experiment overview

The effect of retainability was confirmed on damaged hair bundles treated with the hair improving agents containing the L-ascorbic acid derivatives represented by general formula (1) and salts thereof.

### 2. Experiment method

The hair bundles treated with the products 2-1 to 2-4 and Comparative Product 2-1 in Experiment method of Example 2 were immersed in a 3% by mass sodium lauryl sulfate aqueous solution, washed by swaying up and down, and subsequently immersed in water to rinse by swaying up and down. This procedure was repeated 28 times. The hair bundles obtained by this treatment were measured for the contact angle of water against the hair surface using an automatic contact angle meter (product of Kyowa Interface Science Co., Ltd., DropMaster, DM-501).

### 3. Results

The experiment results are shown in Table 3. The confirmation was made on the water-repellency retainability on the hair surface of hair bundles treated with the product 2-1 for use in the present invention containing one or more selected from the L-ascorbic acid derivatives represented by general formula (1) and salts thereof, and the effect was retained for an even more extended period of time when heat treatment was carried out.

**[Table 3]**

| | Present invention products | | | | | | | | Comparative product | |
|---|---|---|---|---|---|---|---|---|---|---|
| | 2-1 | | 2-2 | | 2-3 | | 2-4 | | 2-1 | |
| Main component used | A | | B | | C | | D | | G | |
| Heat treatment | Not treated | Treated | Not treated | Treated | Not treated | Treated | Not treated | Treated | Not treated | Treated |
| Contact angle (°) | 100 | 114 | 97 | 108 | 94 | 100 | 96 | 102 | 81 | 75 |

| | | | | | | | | | | |
|---|---|---|---|---|---|---|---|---|---|---|
| A: Ascorbyl tetra-2-hexyldecanoate B: Ascorbyl dipalmitate C: Ascorbyl palmitate D: Trisodium ascorbyl palmitate phosphate G: Squalane | | | | | | | | | | |

### Example 4

### Sensory evaluation on damaged hair at different concentrations

### 1. Experiment overview

Sensory evaluation was carried out on damaged hair bundles at different concentrations of the damaged hair improving agents containing the L-ascorbic acid derivatives represented by general formula (1) or salts thereof.

### 2. Experiment method

Using ascorbyl tetra-2-hexyldecanoate, which is the compound selected from the L-ascorbic acid derivatives represented by general formula (1) and salts thereof, as the component used shown in Table 4, and squalane as the oil component for the balance, each of the damaged hair improving agents was prepared in such a way that the concentration of the component used was 1% by mass, 10% by mass, 50% by mass, or 100% by mass (present invention products 4-1 to 4-4). Additionally, squalane (100% by mass) was used as Comparative Product 4-1. Hair bundles made of healthy persons' hair (product of Beaulax Co., Ltd.) were bleached using hydrogen peroxide water and an ammonia aqueous solution to produce hair bundles of damaged hair. After each of the damaged hair improving agents was applied to the damaged hair bundle produced, and was settled throughout the entire damaged hair bundle, no heat treatment was carried out to some of the hair bundles whereas heat treatment was carried out to other hair bundles by blowing hot air for 5 minutes using a blow dryer. Regardless of carrying out heat treatment, the hair bundles were rinsed alternately with a 3% by mass sodium lauryl sulfate aqueous solution and water. This procedure was repeated three times and subsequently the moisture on the hair bundles was wiped off using a towel. Sensory evaluation on the obtained hair bundles was carried out by 5 subjects using the following evaluation criteria.

### 3. Results

The experiment results are shown in Table 4. When the damaged hair improving agents, which are the present invention products 4-1 to 4-4 containing one or more selected from the L-ascorbic acid derivatives represented by general formula (1) and salts thereof, were applied to the damaged hair, improvements in the sensory evaluations on finger runningness, glossy appearance, firmness and body, and manageability were confirmed and further the improving effects were confirmed to have been retained when the amount of the L-ascorbic acid derivatives represented by general formula (1) and salts thereof blended was changed. Furthermore, the improving effects were demonstrated without reduction even when heat treatment was carried out.

**[Table 4]**

| | | | | | | | | | Comparative product | |
|---|---|---|---|---|---|---|---|---|---|---|
| | 4-1 | | 4-2 | | 4-3 | | 4-4 | | 4-1 | |
| Amount of component used blended | 1.0 | | 10.0 | | 50.0 | | 100.0 | | 0.0 | |
| Amount of squalane blended | 99.0 | | 90.0 | | 50.0 | | 0.0 | | 100.0 | |
| Heat treatment | Not treated | Treated | Not treated | Treated | Not treated | Treated | Not treated | Treated | Not treated | Treated |
| Finger runningness | very good | very good | very good | very good | good | good | fair | good | poor | poor |
| Glossy appearance | very good | very good | very good | very good | very good | very good | good | good | poor | poor |
| Manageability | very good | very good | very good | very good | good | very good | good | good | poor | poor |
| Dryness | good | good | good | good | good | good | good | good | poor | poor |
| Firmness and Body | very good | very good | very good | very good | good | good | good | good | poor | poor |

### Evaluation criteria

Notably improved compared to damaged hair before the damaged hair improving agent was applied: Very good
Improved compared to damaged hair before the damaged hair improving agent was applied: Good
Slightly improved compared to damaged hair before the damaged hair improving agent was applied: Fair
No change compared to damaged hair before the damaged hair improving agent was applied: Poor

Hereinbelow, application examples of the damaged hair improving cosmetic in which one or more selected from the L-ascorbic acid derivatives represented by general formula (1) and salts thereof of the present invention are blended. The amounts blended are by % by mass. Examples 5 to 13 were all confirmed to have improving effects on damaged hair by the evaluation methods of Examples 1 to 4.

### Example 5

### Hair oil

| | |
|---|---|
| Ascorbyl tetra-2-hexyldecanoate/squalane solution (5%) | 20.0 (% by mass) |
| Tri(caprylic acid/capric acid)glyceryl | 28.0 |
| Olive oil | 1.0 |
| Tocopherol | 1.0 |
| Perfume | Micro amount |
| Methylheptyl palmitate | Balance |
| Total amount | 100.0 |

Preparation method: Ingredients blended are homogeneously mixed by stirring.

### Example 6

### Hair oil mist

| | |
|---|---|
| Ascorbyl tetra-2-hexyldecanoate | 1.0 (% by mass) |
| Squalane | 10.0 |
| Methylheptyl myristate | 5.0 |
| Perfume | Micro amount |
| Isododecane | Balance |
| Total amount | 100.0 |

Preparation method: Ingredients blended are homogeneously mixed by stirring.

### Example 7 (not invention)

### Hair shampoo

| | |
|---|---|
| (A) Ascorbyl dipalmitate | 1.0 (% by mass) |
| Sodium lauroyl methylaminopropionate aqueous solution (30%) | 20.0 |
| Sodium methyl cocoyl taurate aqueous solution (30%) | 10.0 |
| Cocamidopropyl betaine aqueous solution (15%) | 20.0 |
| Cocamido DEA | 4.0 |
| Polyglyceryl-10 oleate | 1.0 |
| 1,3-Butylene glycol | 3.0 |
| Phenoxyethanol | 0.7 |
| Preservative | Suitable amount |
| (B) Polyquaternium-10 | 0.5 |
| Purified water | Balance |
| Total amount | 100.0 |

Preparation method: Phases A and B are heated to 80°C and homogeneously dissolved, respectively. Further, phase B is added while stirring phase A and cooled down to 30°C.

### Example 8 (not invention)

### Hair conditioner

| | |
|---|---|
| (A) Behenamidopropyl dimethylamine | 2.5 (% by mass) |
| Glyceryl stearate | 1.0 |
| Cetyl ethylhexanoate | 1.0 |
| Squalane | 3.0 |
| Stearyl alcohol | 8.0 |
| Dimethyl silicone | 6.0 |
| Preservative | Suitable amount |
| (B) Trisodium ascorbyl palmitate phosphate | 1.0 |
| Hydroxyethyl cellulose | 0.3 |
| Propylene glycol | 5.0 |
| Preservative | Suitable amount |
| Purified water | Balance |
| Total amount | 100.0 |

Preparation method: Phase A and phase B are respectively heated to 80°C and homogeneously dissolved, and phase A is added while stirring phase B and made homogeneous by stirring. Stirring is continued to cool down to 30°C.

### Example 9 (not invention)

### In-bath treatment

| | |
|---|---|
| (A) Ascorbyl dipalmitate | 1.0 (% by mass) |
| Stearyl alcohol | 8.0 |
| Squalane | 10.0 |
| Preservative | Suitable amount |
| (B) Behentrimonium Chloride | 3.0 |
| Cetrimonium chloride aqueous solution (30%) | 3.3 |
| Dipropylene glycol | 5.0 |
| Hydroxyethyl cellulose aqueous solution (1%) | 30.0 |
| Phenoxyethanol | 0.4 |
| Purified water | Balance |
| Total amount | 100.0 |

Preparation method: Phase A and phase B are heated to 80°C and homogeneously dissolved. Phase A is added while stirring phase B, and stirring is continued to cool down to 30°C.

### Example 10 (not invention)

### Hair milk

| | |
|---|---|
| (A) Ascorbyl palmitate | 0.5 (% by mass) |
| Ascorbyl tetra-2-hexyldecanoate | 0.5 |
| Tri(caprylic acid/capric acid)glyceryl | 2.0 |
| Cetyl ethylhexanoate | 3.0 |
| PEG-40 hydrogenated castor oil | 0.5 |
| (B) Propane diol | 3.0 |
| Acrylates/(C10-30) alkyl acrylate crosspolymer aqueous solution (1%) | 40.0 |
| Ethanol | 10.0 |
| Phenoxyethanol | 0.5 |
| Arginine | 0.4 |
| Purified water | Balance |
| Total amount | 100.0 |

Preparation method: Phase A and phase B are heated to 80°C and homogeneously dissolved. Phase A is added to phase B and emulsified at 80°C. Stirring is continued to cool down to 30°C.

### Example 11 (not invention)

### Hair treatment

| (A) Ascorbyl stearate | | 1.0 (% by mass) |
|---|---|---|
| | Stearamidopropyl dimethylamine | 2.5 |
| | Glyceryl stearate | 0.5 |
| | Cetyl alcohol | 2.0 |
| | Polyglyceryl-10 (dimer dilinoleate/stearate/ hydroxystearate) | 3.0 |
| | Isopropyl palmitate | 3.0 |
| | Propylene glycol laurate | 1.0 |

| (B) Polyquaternium-10 | | 0.5 |
|---|---|---|
| | 1,3-Butylene glycol | 10.0 |
| | Glutamic acid | 0.8 |
| | Preservative | Micro amount |
| | Purified water | Balance |
| | Total amount | 100.0 |

Preparation method: Phase A and phase B are heated to 80°C and homogeneously dissolved. Phase A is added to phase B and emulsified at 80°C. Stirring is continued to cool down to 30°C.

### Example 12

### Leave-on treatment

| (A) Ascorbyl tetra-2-hexyldecanoate | | 1.0 (% by mass) |
|---|---|---|
| | Behenyl alcohol | 1.0 |
| | Glyceryl stearate | 0.5 |
| | Stearyl alcohol | 1.0 |
| | Cetearyl alcohol | 1.5 |
| | Preservative | Micro amount |
| (B) Pentylene glycol | | 2.0 |
| | 1,3-Buthylene glycol | 5.0 |
| | Glycerin | 1.0 |
| | Preservative | Micro amount |
| | Purified water | Balance |
| | Total amount | 100.0 |

Preparation method: Phase A and phase B are homogeneously heated, phase A is added to phase B and emulsified.

Stirring is continued to cool down to 30°C.

### Example 13

### Hair oil

| | |
|---|---|
| Ascorbyl tetra-2-hexyldecanoate/squalane solution (30%) | 3.5 (% by mass) |
| Methylheptyl laurate | 48.0 |
| Apricot kernel oil | 2.0 |
| Tocopherol | 1.0 |
| Perfume | Micro amount |
| Dimethyl silicone | Balance |
| Total amount | 100.0 |

Preparation method: Blending ingredients are homogeneously mixed by stirring.

### Industrial Applicability

The present invention has excellent damage care effects on damaged hair and thus can be used for hair cosmetics.

## Claims

1. Use of a damaged hair improving agent comprising, as a main component, one or more selected from L-ascorbic acid derivatives represented by the following general formula (1) and salts thereof: 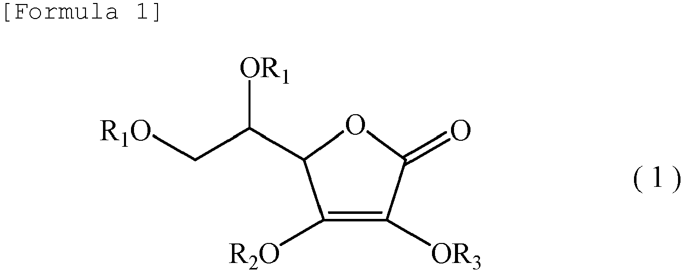
wherein R₁ is each independently a linear or branched saturated or unsaturated fatty acid residue having 8 to 36 carbon atoms, R₂ is a linear or branched saturated or unsaturated fatty acid residue having 8 to 36 carbon atoms, R₃ is a linear or branched saturated or unsaturated fatty acid residue having 8 to 36 carbon atoms
wherein this derivative is present in an amount of from 0.5 to 20% by mass;
for increasing the hydrophobicity of the hair surface and/or making hair smooth and/or further enhancing glossiness.

## Patentansprüche

1. Verwendung von einem Mittel zur Verbesserung von geschädigtem Haar, das als Hauptkomponente ein oder mehrere Derivate der L-Ascorbinsäure der folgenden allgemeinen Formel (1) und deren Salze enthält: 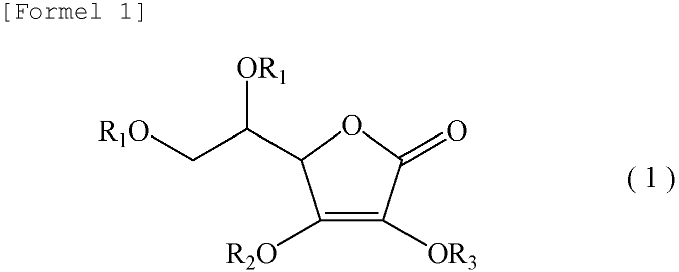
worin R₁ jeweils unabhängig voneinander ein linearer oder verzweigter gesättigter oder ungesättigter Fettsäurerest mit 8 bis 36 Kohlenstoffatomen ist, R₂ ein linearer oder verzweigter gesättigter oder ungesättigter Fettsäurerest mit 8 bis 36 Kohlenstoffatomen ist, R₃ ein linearer oder verzweigter gesättigter oder ungesättigter Fettsäurerest mit 8 bis 36 Kohlenstoffatomen ist,
wobei dieses Derivat in einer Menge von 0,5 bis 20 Gew.-% vorhanden ist,
um die Hydrophobie der Haaroberfläche zu erhöhen und/oder das Haar geschmeidig zu machen und/oder den Glanz zu verstärken.

## Revendications

1. Utilisation d'un agent améliorant les cheveux abîmés comprenant, comme un composant principal, un ou plusieurs dérivés de l'acide L-ascorbique représentés par la formule générale suivante (1) et leurs sels : 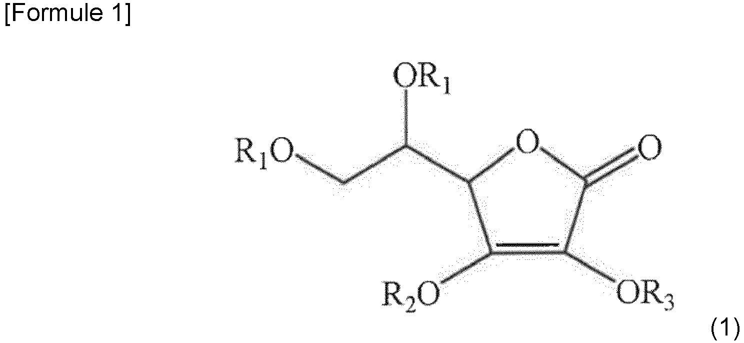
dans laquelle R₁ est chacun indépendamment un résidu d'acide gras saturé ou insaturé, linéaire ou ramifié, ayant 8 à 36 atomes de carbone, R₂ est un résidu d'acide gras saturé ou insaturé, linéaire ou ramifié, ayant 8 à 36 atomes de carbone, R₃ est un résidu d'acide gras saturé ou insaturé, linéaire ou ramifié, ayant 8 à 36 atomes de carbone
dans laquelle ce dérivé est présent en une quantité allant de 0,5 à 20 % en masse ;
pour augmenter l'hydrophobicité de la surface de cheveux et/ou rendre des cheveux lisses et/ou améliorer encore la brillance.
